# EUROPEAN PATENT SPECIFICATION

(11) **EP 1 467 895 B1**
(45) Date of publication and mention of the grant of the patent: **09.11.2005**
(21) Application number: 02804643.1
(22) Date of filing: 11.12.2002
(51) Int. Cl.: B60R 21/01, A61N 1/36

(54) **ELECTROSTIMULATION DEVICE FOR USERS OF MEANS OF TRANSPORT**
ELEKTROSIMULATIONSVORRICHTUNG FÜR BENUTZER VON TRANSPORTMITTELN
DISPOSITIF D'ELECTROSTIMULATION POUR UTILISATEURS DE MOYENS DE TRANSPORT

(30) Priority: 12.12.2001 IT UD20010206
(43) Date of publication of application: 20.10.2004
(73) Proprietor: Universita' Degli Studi di Udine, 33100 Udine (IT)
(72) Inventor: PASCOLO, Paolo, I-33100 Udine (IT); PASCOLO, Carlo, I-33100 Udine (IT)
(74) Representative: Petraz, Gilberto Luigi
(86) International application number: PCT/IB2002/005274
(87) International publication number: WO 2003/049976

(56) References cited:
- EP-A- 0 339 665
- WO-A-00/71075
- US-B1- 6 189 969
- PHEN R L ET AL: "Advanced Air Bag Technology Assessment - Final Report" JET PROPULSION LAB PROGRESS REPORT, XX, XX, April 1998 (1998-04), XP002137509

## Description

### FIELD OF THE INVENTION

The present invention concerns an electrostimulation device which can be used to prevent physical damage to users of a means of transport, whether it be a vehicle used on the ground, a boat or an aircraft. The electrostimulation device according to the invention is able to transmit at least an electric impulse to one or more parts of the body of a user when there is a sudden acceleration or deceleration of the means of transport on which the user is travelling, such as when it suffers a sudden impact, or in the event that the user is ejected from the cockpit in the case of an aircraft, or in other similar or comparable cases.

The device according to the present invention is able to cause a localized contraction of the muscles of part of the user's body almost instantaneously by means of said electric impulse and in this way, in the event of impacts and knocks and/or other high acceleration stresses (high number of g where g = gravity acceleration), prevents and/or reduces traumas of the body, such as those deriving for example from the whiplash effect.

### BACKGROUND OF THE INVENTION

The Applicant is not aware of devices installed on board a means of transport which can be used to protect and safeguard drivers and passengers of vehicles from the damage caused, for example, by the so-called whiplash effect, which a human being suffers as a consequence of a violent counter-blow due to a sudden and unexpected acceleration or deceleration of the vehicle, (an automobile, an aircraft, a boat, a train, a bus or suchlike), for example in the case of an impact in an accident, or when the user is ejected from a cockpit of an aircraft, or because of other manoeuvres, for example in military aircraft, which entail violent variations in speed.

In the specific case of an automobile, there are known, for example from the article by R.L. Phen et al, "Advanced Air bag Technology Assessment - Final Report", devices to protect the users, such as airbags, which in any case do not help to protect the person from the whiplash effect but which serve to prevent or reduce the impact of the driver or passenger against parts of the driver's compartment of the vehicle in the event of a crash.

Moreover the reaction times of a human being (psycho-technical reaction time) in the case of a crash are, at best, in the range of 3 tenths of a second, while the average value is in the range of 0.765 seconds. These values are high and altogether insufficient to react against an unexpected and potentially dangerous event. For this reason the human being cannot avoid considerable damage and traumas like those caused, for example, by the whiplash effect, in the case of a violent impact or crash or sudden accelerations.

The Applicant has devised and embodied the present invention to overcome this shortcoming and to obtain further advantages.

### SUMMARY OF THE INVENTION

The present invention is set forth and characterized essentially in the main claim, while the dependent claims describe other innovative characteristics of the invention.

The purpose of the invention is to achieve an electrostimulation device which can be used to protect and/or safeguard drivers and passengers in vehicles, trains, aircraft, boats or other moving means such as the most recent fairground attractions where the user is subjected to violent accelerations and decelerations.

In accordance with this purpose, an electrostimulation device to prevent damage to users of a means of transport according to the present invention comprises sensor means able to detect anomalous variations in particular parameters of the conditions of use of the means of transport, and stimulation means governed by such sensor means and associated with one or more particular and localized parts of the body of the users of the means of transport.

In a preferential embodiment, the device according to the present invention also comprises a processing and control unit which is able to process the signals generated by the sensor means and to generate correlated stimulation signals for one or more of said stimulation means.

The means of transport in which the device according to the present invention can be used are vehicles such as automobiles, trucks, coaches, etc., motor vehicles or other individual or collective means of transport such as trains or planes, both civilian and military.

In the functioning of the device, the sensor means are connected to the processing and control unit from which one or more electrodes branch off. The electrodes are positioned in such a manner as to selectively excite defined parts of the muscles of parts of the human body such as for example the head, neck, trunk, limbs or other parts, so as to determine an almost instantaneous contraction thereof as a consequence of a sudden impact detected by the sensor means and/or because of stresses of other types due to a high acceleration or deceleration. Such contraction allows the user to be prepared, for example, for a sudden variation in the speed of the means of transport in which he is travelling so as to minimize the consequences of the traumatic incident.

It has been proved in experiments that, given the same variation in speed, a suitably stimulated person can reduce the oscillations of the head up to 70%, and hence the state of stress of the cervical rachis compared to the same person who has not been electrostimulated.

The action of the electrostimulation device, in some cases, can also partly compensate for the variation in the range of the blood pressure.

The sensor means can be distributed in different parts of the means of transport: for example, in the case of an automobile, they can be inserted in the seat, the dashboard, or in the front or rear part of the body work, whereas in the case of motorcycles they can be inserted in the motorcycle itself and/or in the rider's helmet.

In the case of aircraft, apart from the positions mentioned above, the sensor means can be inserted into the nacelle, or associated with various members to pilot, or to eject the pilot from, the aircraft.

The sensor means can be of various shape and are connected, by means of cables and connectors, to the processing and control unit and by means of this to the stimulation means.

According to a variant, the connection between the sensor means and the processing and control unit and/or between the latter and the stimulation means occurs via radio, by means of ultrasounds or infra-red rays.

The sensor means are of the type able to detect a sudden variation in the conditions of use of the means of transport and can comprise for example accelerometers, inclinometers and sensors of other types. The sensor means are able to send a signal to the processing and control unit to activate the stimulation electrodes, with a consequent emission of an electric impulse which acts locally on the nervous system and on the muscles of the user.

In a preferential form of embodiment the processing and control unit comprises at least a programmable microprocessor.

In this preferential form of embodiment the sensor means can easily be interfaced, for example in the case of automobiles, with those of a device to activate at least an airbag.

The stimulation means can be positioned according to a variable mapping depending on the type of user, to take into account individual variability. In the simplest cases, when the paravertebral muscles are excited, the stimulation means comprise electrodes which are positioned in a variable manner. For example there can be from two to sixteen electrodes and hence from one to eight channels. In a particular case, there may be a single outlet with two electrodes. In the particular case of aircraft, there may be more than 16 electrodes, and their number will depend substantially on the parts of the body which are to be stimulated.

The positioning of the stimulation electrodes depends on which muscles are to be excited, for example the rear muscles of the head, the neck and the shoulders, the trunk and the limbs, according to the type of situation and problem to be dealt with.

In a preferential embodiment, the electrodes are distributed so that they start the stimulation below the C3 vertebra in order to prevent influence on the brain (electric shock).

In another preferential embodiment, the electrodes are distributed so that they stimulate the neck muscles in correspondence, for example, with the center line of the backbone for a maximum width of 3.5ö4 cm between vertebra C3 and C7.

In another preferential embodiment, the electrodes are distributed so that they stimulate the shoulder muscles from vertebra C7 to T1 and comprise the middle trapezius muscle for the whole width of the shoulders.

For other purposes, for example to control the range of the blood pressure, the electrodes can be positioned so as to stimulate all or a large part of the muscles.

According to the type of compartment and the type of stress which is to be reduced or from which the driver or passenger is to be protected, for example head-on and/or rear impact rather than a fall from a motorcycle or a simple whiplash, the electrodes can be positioned differently on the person and using different methods.

According to further variants, the electrodes can be inserted according to the vehicle, where the device according to the present invention is used, in rear pads of a helmet, in a collar, in soft, hidden fork-shaped supports in the head-rest of an automobile, on the arms of glasses or also in metal extensions or branches integrated into the head-rest or in the cushion associated with a seat, or suchlike.

In the preferential embodiment of the invention, the impulses which can be applied are variable in amplitude, width, frequency of application, with or without the negative impulse.

Preferential values of the electric impulses generated are generally within a difference in potential of between 50 ö 250 V, with a frequency of between 15 ö 170 Hz, with a width of between 10 ö 400 µs and a current intensity of between 1 ö 100 mA.

### BRIEF DESCRIPTION OF THE DRAWINGS

These and other characteristics of the invention will be apparent from the following description of a preferential form of embodiment, given as a non-restrictive example, with reference to the attached drawings wherein:
- fig. 1 is a schematic view of a means of transport wherein an electrostimulation device according to the invention in installed according to one embodiment;
- fig. 2 shows a variant of fig. 1;
- fig. 3 is a schematic view of an electrostimulation device as shown in fig. 2 applied in another means of transport;
- fig. 4 is a block diagram of the processing and control unit contained in the device shown in fig. 1;
- fig. 5 is a schematic view of the electrostimulation device in fig. 1 applied in an aircraft.

### DETAILED DESCRIPTION OF SOME PREFERENTIAL FORMS OF EMBODIMENT OF THE INVENTION

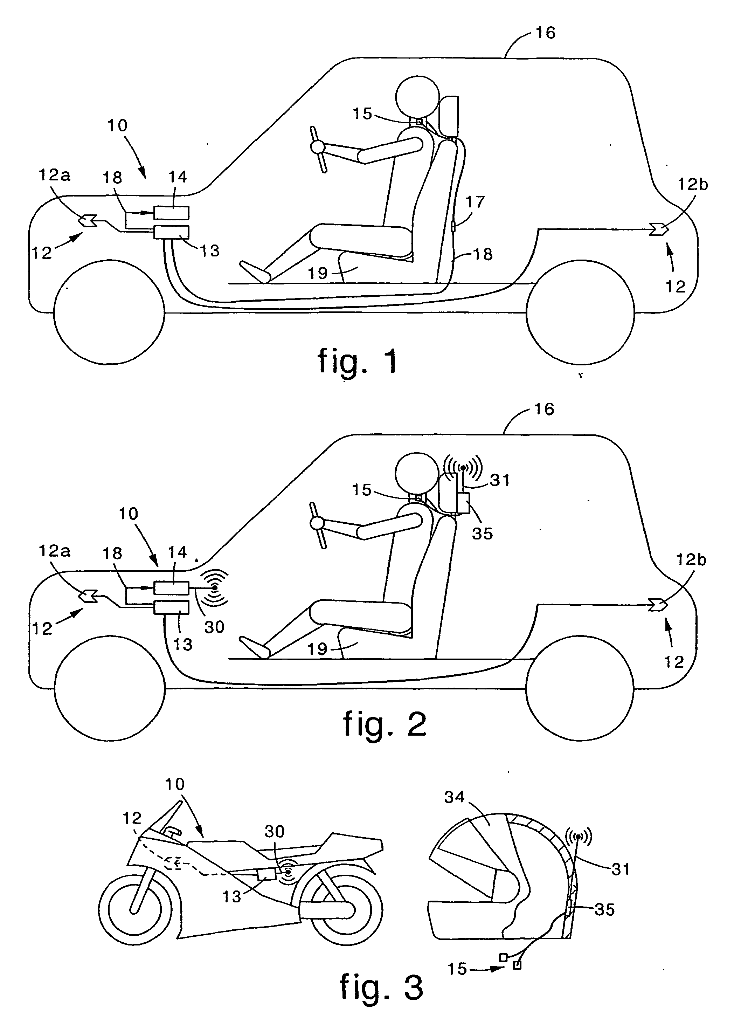

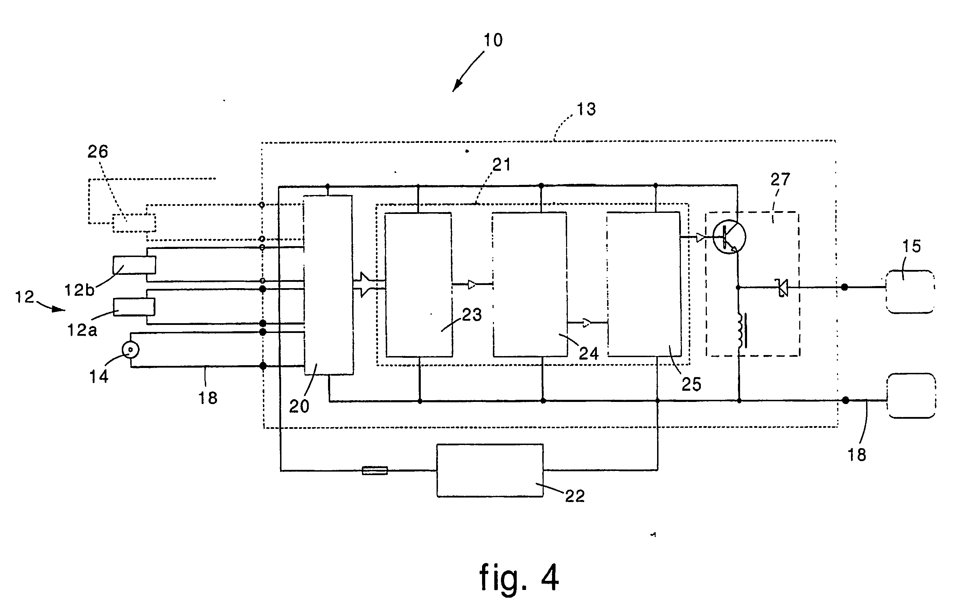

With reference to figs. 1 and 4, an electrostimulation device 10 according to the present invention comprises a processing and control unit 13, and is connected to and installed in an automobile 16.

The electrostimulation device 10 is able to prevent the so-called whiplash effect to a user in the event of a possible accident, impact or sudden acceleration/deceleration of another type. The device 10 comprises in its essential parts sensor elements 12 consisting, in this case, of a first accelerometer 12a installed in the front part of the automobile 16, and a second accelerometer 12b installed in the rear part, an airbag unit 14, the processing and control unit 13 (shown in detail in fig. 4), a feeder unit 22 and a pair of electrodes 15, mounted on a collar suitable to keep the electrodes 15 stably in contact on the user's head.

The first and second accelerometer 12a and 12b are positioned to respectively detect sudden accelerations or decelerations caused by impacts from the front or rear.

It is clear that the use of accelerometers 12a and 12b is only an example, and any type of sensor 12 suitable for the purpose can be used, for example an inclinometer or other type of analogous or comparable sensor. It is also clear that the representation of the electrodes mounted on the collar is an example, and can be replaced, according to a variant, by a helmet 34 (fig. 3), by fixed electrodes mounted on the head-rest, on the seat, or on another part of the automobile 16 or by any other configuration suitable for the purpose.

In the embodiment shown in fig. 1, each of the electrodes 15 is fixed to the end of connection cables 18 and is connected by a connector 17 to the processing and control unit 13 located in the front portion of the automobile 16, in this case in front of the driver's position. The pair of electrodes 15, thanks to the cables 18, can be applied to the neck of a user driving on the seat 19 of the automobile 16.

According to a variant not shown in the drawing, the processing and control unit 13 is connected by means of connection cables 18 and the connector 17 also to other pairs of electrodes 15 suitable to reach any one of the seats of the automobile 16.

The first accelerometer 12a and the second accelerometer 12b are sensors to detect accelerations (positive or negative), for example of the monoaxial type model M352C68 by PCB Piezotronics, which emits a tension signal when it detects an acceleration.

The processing and control unit 13 is able to evaluate the acceleration threshold and then establishes whether to generate an activation signal or not.

The processing and control unit 13 shown in figs. 1-2 is connected by means of electric connection cables 18 to the first and second accelerometer 12a, 12b and to the device to activate the airbag 14, of a conventional type.

With reference to fig. 4, the accelerometers 12a, 12b and the system to activate the airbag 14 are connected to the terminals at inlet to the processing and control unit 13.

According to a variant, the processing and control unit 13 is independent from the device to activate the airbag 14.

According to another variant, the terminals at inlet to the processing and control unit 13 are connected to other sensors, indicated by a line of dashes in fig. 4, for example inclinometers 26.

In this form of embodiment the processing and control unit 13 comprises a first reception and adaptation block 20 by means of which the electric signal arriving from the various sensors (for example 12a, 12b, 26...) connected is transmitted to a programmable microprocessor 21.

The block 20 and/or the programmable microprocessor 21 can be regulated so that an electric signal arriving from a sensor 12, or group of sensors 12, is dominant with respect to another.

The programmable microprocessor 21 comprises, in its essential parts, developed through processing, an AND/OR logical matrix 23, a timer block 24 and an impulse generator block 25.

The signals arriving from the accelerometers 12a, 12b and/or the signal arriving from the unit to activate the airbag 14, in the event of a sudden deceleration or an impact from front or rear, are recognized and validated by the programmable microprocessor 21, in correspondence with the AND/OR logical matrix 23. The latter is suitable to activate the timer block 24 which is able to determine the time for which electrostimulation has to be applied to the user, in response to the stress detected by the accelerometers 12a, 12b.

The timer block 24 then activates the impulse generator 25 which provides to make the outlet power unit 27 produce the stimulation current.

In this way the cable connection 18 carries the stimulation impulses to the two electrodes 15 inserted in the collar.

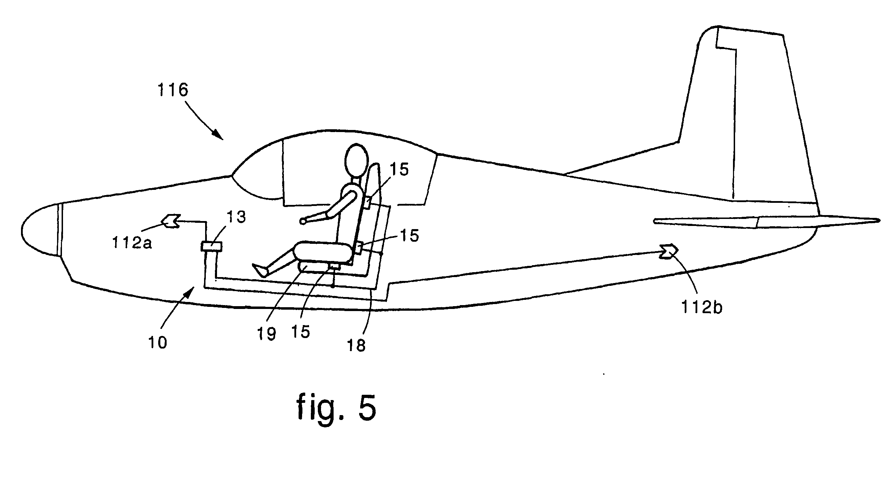

In the embodiment shown in fig. 5, the electrostimulation device 10 as described heretofore is installed in an aircraft 116 equipped with a seat 19 associated with ejection means. In this case a plurality of electrodes 15 are associated with the seat 19 and act in cooperation with various parts of the pilot's body, to prevent any damage deriving from the violent acceleration caused by the ejection of the seat 19, or by other anomalous situations which can occur, for example during take-off, landing or otherwise. In the case shown schematically here, a first sensor 112a and a second sensor 112b are arranged in different zones of the aircraft 116, to detect situations which cause sudden accelerations or decelerations to which the pilot or a possible passenger is subject.

For example, a first sensor 112a can be associated directly with the seat 19 and can detect when the ejector mechanism is engaged, and send the relative signal to the processing and control unit 13 in order to activate the electrodes 15 which perform the electrostimulation. The second sensor 112b can be located at any point of the nacelle, and can be an accelerometer or inclinometer or a sensor of another type, which detects anomalous situations concerning the functioning of the aircraft, for example a crash, a sudden drop in altitude, a violent inclination, a sudden acceleration or other problem.

According to a further variant indicated in figs. 2-3, the processing and control unit 13 comprises a signal transmission circuit via radio and a transmission antenna 30. The electrodes 15, connected to a device 35 comprising an antenna 31 to receive the radio signal and at least a signal converter and an impulse generator, are positioned on a seat 19 of the automobile 16 in fig. 2 or on a helmet 34 in fig. 3.

In this way the connection between the processing and control unit 13 and the electrodes 15 occurs via radio, so as to reduce the number of cables between the processing and control unit 13 and the points where the electrodes 15 are positioned.

It is clear however that modifications and/or additions of parts can be made to the device 10 as described heretofore without departing from the field and scope of the present invention.

For example the circuit which comprises the processing and control unit 13, instead of comprising a programmable microprocessor 21, can simply be made with elements of a traditional electric or electronic type.

Further, in another embodiment, the stimulation means can comprise not electrodes 15 provided in continuous contact with parts of the user's body, but they can consist of delivering means for delivering a conductive fluid, i.e. clouds and/or sprays of a conductive gas, or jets of liquid, also nebulized, having property of electric conduction, such as saltwater or like.

In the same manner as above for the solution with the electrodes 15, the stimulation means are activated by the detection of a sudden acceleration/deceleration in order to deliver such a conductive fluid towards one or more parts of the body, activating in the user the above described electrostimulation mechanism of the muscles.

The delivering means can be mounted on any suitable position of the vehicle or of the user's equipment, for example installed and/or integrated in the seat 19, in a head-rest, in a helmet 34, in a part of the body work, or of the dashboard, or others.

## Claims

1. Electrostimulation device to prevent damage and traumas to users of a means of transport (16), comprising sensor means (12a, 12b, 112a, 112b, 26) able to detect variations in particular parameters of the conditions of use of said means of transport (16), **characterized in that** it comprises stimulation means (15) able to be associated with particular parts of the body of the users of said means of transport (16) and governed by said sensor means (12a, 12b, 112a, 112b, 26) in order to generate associated signals to stimulate said parts of the body of the user in response to the detection of anomalous situations in the parameters of functioning of said means of transport (16) as detected by said sensor means (12a, 12b, 112a, 112b, 26).

2. Electrostimulation device as in claim 1, **characterized in that** it comprises a processing and control unit (13) able to receive and process the signals transmitted by said sensor means (12a, 12b, 112a, 112b, 26) and to condition the activation of said stimulation means (15) in correlated manner.

3. Electrostimulation device as in claim 2, **characterized in that** said processing and control unit (13) is suitable to activate said stimulation means (15) by means of an electric signal transmitted through cables and connectors (17, 18).

4. Electrostimulation device as in claim 2, **characterized in that** said processing and control unit (13) comprises transmission means (30) to transmit radio waves and said stimulation means (15) are associated with reception means (31) to receive said radio waves.

5. Electrostimulation device as in any claim hereinbefore, **characterized in that** said means of transport (16) comprises an automobile, a motor vehicle, or other means able to transport persons individually or collectively, such as trains or planes, civilian or military.

6. Electrostimulation device as in any claim hereinbefore, **characterized in that** said anomalous situation comprises a sudden variation in speed, either positive or negative, or in inclination of said means of transport (16).

7. Electrostimulation device as in any claim hereinbefore, **characterized in that** said stimulation means comprise at least an electrode (15) able to be applied on at least a localized part of the body of a user in order to transmit at least an electric impulse to said part.

8. Electrostimulation device as in claim 7, **characterized in that** there are at least two electrodes (15) to define at least a corresponding channel to transmit said electric impulse.

9. Electrostimulation device as in claim 8, **characterized in that** there are 16 or more electrodes (15).

10. Electrostimulation device as in any claim hereinbefore, **characterized in that** said stimulation means (15) comprise a single outlet with two electrodes.

11. Electrostimulation device as in any claim hereinbefore, **characterized in that** said stimulation means (15) are distributed in such a manner as to stimulate at least part of the paravertebral muscles of the head, of the neck and of the shoulders of said user.

12. Electrostimulation device as in any claim hereinbefore, **characterized in that** said stimulation means (15) are distributed in such a manner as to stimulate the part of the user's muscles which can affect the range of the blood pressure.

13. Electrostimulation device as in any claim hereinbefore, **characterized in that** said stimulation means (15) are inserted in the rear pads of a protection helmet (34), in a collar, in a seat (19) or in soft, hidden fork-shaped supports of a head-rest in said means of transport (16) or on the arms of the glasses of said user.

14. Electrostimulation device as in any claim from 7 to 13 inclusive, **characterized in that** said electric impulse is variable in amplitude, width, frequency of application, with or without the presence of the negative impulse.

15. Electrostimulation device as in claim 14, **characterized in that** said electric impulse has a difference in potential of between 50 and 250 V, a frequency of between 15 and 170 Hz, a width of between 10 and 400 µs and a current intensity of between 1 and 100 mA.

16. Electrostimulation device as in any claim hereinbefore, **characterized in that** said sensor means (12a, 12b, 112a, 112b, 26) are able to be interfaced in said means of transport (16) with sensor means associated with a device to activate at least an airbag (14) or at least an ejector seat (19).

17. Electrostimulation device as in any claim hereinbefore, **characterized in that** said sensor means (12a, 12b, 112a, 112b, 26) are able to be positioned in a seat (19) or on the dashboard of a vehicle, in the nacelle or associated with members to pilot an aircraft (116).

18. Electrostimulation device as in any claim hereinbefore, **characterized in that** said sensor means (12a, 12b, 112a, 112b, 26) are able to be positioned in a seat of a motorcycle and/or in association with a rider's helmet.

19. Electrostimulation device as in any claim from 2 to 18 inclusive, **characterized in that** said processing and control unit (13) comprises at least a programmable microprocessor (21).

20. Electrostimulation device as in claim 19, **characterized in that** said programmable microprocessor (21) comprises a processing logical matrix (23), a timer block (24) and an impulse generating block (25, 27), associated with said stimulation means (15).

21. Electrostimulation device as in any claim from 1 to 6 inclusive, **characterized in that** said stimulation means comprise delivering means able to deliver a conductive fluid towards one or more parts of the user's body.

22. Electrostimulation device as in claim 21, **characterized in that** said conductive fluid comprises a conductive gas or liquid, such as saltwater or like.

23. Electrostimulation device as in claim 22, **characterized in that** said conductive liquid is delivered in a nebulized form.

24. Electrostimulation device as in claim 21, **characterized in that** said delivering means are installed and/or integrated in a part of the vehicle or of the user's equipment, such as the seat, the head-rest, the helmet, the body work, the dashboard, or others.

## Patentansprüche

1. Elektrostimulationsvorrichtung, um eine physische Beeinträchtigung und um Traumen bei Nutzern von einem Transportmittel (16) zu verhindern, welche Sensorelemente (12a, 12b, 112a, 112b, 26) umfasst, welche Veränderungen bei bestimmten Parametern des Betriebszustandes des genannten Transportmittels (16) erkennen können, **dadurch gekennzeichnet, dass** sie Stimulationsmittel (15) umfasst, welche mit bestimmten Körperbereichen der Nutzer des genannten Transportmittels (16) verbunden werden können, und welche durch die genannten Sensorelemente (12a, 12b, 112a, 112b, 26) gesteuert werden, um zugehörige Signale zu generieren, um die genannten Körperbereiche des Nutzers als Reaktion auf das Erkennen von abnormen Zuständen bei den Betriebsparametern des genannten Transportmittels (16) zu stimulieren, welche durch die genannten Sensorelemente (12a, 12b, 112a, 112b, 26) erkannt werden.

2. Elektrostimulationsvorrichtung gemäß Anspruch 1, **dadurch gekennzeichnet, dass** sie eine Verarbeitungs- und Steuereinheit (13) umfasst, welche in der Lage ist, die Signale zu empfangen und zu verarbeiten, welche durch die genannten Sensorelemente (12a, 12b, 112a, 112b, 26) übertragen werden, und welche in der Lage ist, die Aktivierung des genannten Stimulationsmittels (15) in einer entsprechenden Art und Weise vorzunehmen.

3. Elektrostimulationsvorrichtung gemäß Anspruch 2, **dadurch gekennzeichnet, dass** die genannte Verarbeitungs- und Steuereinheit (13) in der Lage ist, das genannte Stimulationsmittel (15) mittels eines elektrischen Signals zu aktivieren, welches über Kabel und Steckverbindungen (17, 18) übertragen wird.

4. Elektrostimulationsvorrichtung gemäß Anspruch 2, **dadurch gekennzeichnet, dass** die genannte Verarbeitungs- und Steuereinheit (13) ein Übertragungsmittel (30) umfasst, um Funkwellen zu übertragen, und das genannte Stimulationsmittel (15) mit dem Empfangsmittel (31) verbunden ist, um die genannten Funkwellen zu empfangen.

5. Elektrostimulationsvorrichtung gemäß einem vorherigen Anspruch, **dadurch gekennzeichnet, dass** das genannte Transportmittel (16) ein Automobil, ein Kraftfahrzeug, oder ein anderes Transportmittel umfasst, welche in der Lage sind, Personen einzeln oder gemeinsam zu transportieren, wie zum Beispiel Züge oder Flugzeuge, zivil oder militärisch.

6. Elektrostimulationsvorrichtung gemäß einem vorherigen Anspruch, **dadurch gekennzeichnet, dass** die genannte abnorme Situation eine plötzliche Veränderung bei der Geschwindigkeit umfasst, entweder positiv oder negativ, oder bei der Neigung des genannten Transportmittels (16).

7. Elektrostimulationsvorrichtung gemäß einem vorherigen Anspruch, **dadurch gekennzeichnet, dass** das genannte Stimulationsmittel mindestens eine Elektrode (15) umfasst, welche auf mindestens einem örtlich eingrenzten Körperbereich eines Nutzers angebracht werden kann, um mindestens einen elektrischen Impuls auf den genannten Bereich zu übertragen.

8. Elektrostimulationsvorrichtung gemäß Anspruch 7, **dadurch gekennzeichnet, dass** mindestens zwei Elektroden (15) vorhanden sind, um mindestens einen entsprechenden Kanal zu definieren, um den genannten elektrischen Impuls zu übertragen.

9. Elektrostimulationsvorrichtung gemäß Anspruch 8, **dadurch gekennzeichnet, dass** 16 oder mehr Elektroden (15) vorhanden sind.

10. Elektrostimulationsvorrichtung gemäß einem vorherigen Anspruch, **dadurch gekennzeichnet, dass** das genannte Stimulationsmittel (15) einen einzigen Ausgang mit zwei Elektroden umfasst.

11. Elektrostimulationsvorrichtung gemäß einem vorherigen Anspruch, **dadurch gekennzeichnet, dass** die genannten Stimulationsmittel (15) derart verteilt sind, um mindestens einen Bereich der paravertebralen Muskeln des Kopfes, des Halses und der Schultern des genannten Nutzers zu stimulieren.

12. Elektrostimulationsvorrichtung gemäß einem vorherigen Anspruch, **dadurch gekennzeichnet, dass** die genannten Stimulationsmittel (15) derart verteilt sind, um den Muskelbereich des Nutzers zu stimulieren, welcher den Blutdruckbereich beeinflussen kann.

13. Elektrostimulationsvorrichtung gemäß einem vorherigen Anspruch, **dadurch gekennzeichnet, dass** das genannte Stimulationsmittel (15) in die hinteren Polster eines Schutzhelmes (34), in ein Halsband, in einen Sitz (19) oder in weiche, verborgene gabelförmige Halterungen einer Kopfstütze in dem genannten Transportmittel (16) eingeführt wird, oder auf die Brillenbügel des genannten Nutzers.

14. Elektrostimulationsvorrichtung gemäß einem der Ansprüche 7 bis 13, **dadurch gekennzeichnet, dass** der genannte elektrische Impuls hinsichtlich Amplitude, Länge und Frequenz der Anwendung variabel ist, mit oder ohne Präsenz eines negativen Impulses.

15. Elektrostimulationsvorrichtung gemäß Anspruch 14, **dadurch gekennzeichnet, dass** der genannte elektrische Impuls eine Spannungsdifferenz zwischen 50 und 250 V aufweist, eine Frequenz zwischen 15 und 170 Hz, eine Länge zwischen 10 und 400 µs und eine Stromstärke zwischen 1 und 100 mA.

16. Elektrostimulationsvorrichtung gemäß einem vorherigen Anspruch, **dadurch gekennzeichnet, dass** die genannten Sensorelemente (12a, 12b, 112a, 112b, 26) in dem genannten Transportmittel (16) angeschlossen werden können, wobei die Sensorelemente mit einer Vorrichtung verbunden sind, um mindestens einen Airbag (14) oder mindestens einen Schleudersitz (19) zu aktivieren.

17. Elektrostimulationsvorrichtung gemäß einem vorherigen Anspruch, **dadurch gekennzeichnet, dass** die genannten Sensorelemente (12a, 12b, 112a, 112b, 26) in einem Sitz (19) oder auf dem Armaturenbrett eines Fahrzeuges, in einer Flugzeugzelle oder verbunden mit Elementen, um ein Flugzeug (116) zu steuern, positioniert werden können.

18. Elektrostimulationsvorrichtung gemäß einem vorherigen Anspruch, **dadurch gekennzeichnet, dass** die genannten Sensorelemente (12a, 12b, 112a, 112b, 26) in einem Motorradsitz und/oder in Verbindung mit dem Helm des Motorradfahrers positioniert werden können.

19. Elektrostimulationsvorrichtung gemäß einem der Ansprüche 2 bis 18, **dadurch gekennzeichnet, dass** die genannte Verarbeitungs- und Steuereinheit (13) mindestens einen programmierbaren Mikroprozessor (21) umfasst.

20. Elektrostimulationsvorrichtung gemäß Anspruch 19, **dadurch gekennzeichnet, dass** der genannte programmierbare Mikroprozessor (21) eine Verarbeitungslogikmatrix (23), einen Timerblock (24) und einen Impulsgeneratorblock (25, 27) umfasst, verbunden mit dem genannten Stimulationsmittel (15).

21. Elektrostimulationsvorrichtung gemäß einem der Ansprüche 1 bis 6, **dadurch gekennzeichnet, dass** das genannte Stimulationsmittel ein Fördermittel umfasst, welches in der Lage ist, ein leitfähiges Fluid zu einem Körperbereich oder zu mehreren Körperbereichen des Nutzers zu fördern.

22. Elektrostimulationsvorrichtung gemäß Anspruch 21, **dadurch gekennzeichnet, dass** das genannte leitfähige Fluid ein leitfähiges Gas oder eine leitfähige Flüssigkeit umfasst, wie zum Beispiel Salzwasser oder dergleichen.

23. Elektrostimulationsvorrichtung gemäß Anspruch 22, **dadurch gekennzeichnet, dass** die genannte leitfähige Flüssigkeit in einer zerstäubten Form gefördert wird.

24. Elektrostimulationsvorrichtung gemäß Anspruch 21, **dadurch gekennzeichnet, dass** das genannte Fördermittel in einem Fahrzeugteil oder in einem Ausrüstungsgegenstand des Nutzers installiert ist und/oder integriert ist, wie zum Beispiel in einem Sitz, in einer Kopfstütze, in einem Helm, in einer Karosserie, in einem Armaturenbrett, oder in anderen Gegenständen.

## Revendications

1. Un dispositif d'électrostimulation pour empêcher les utilisateurs de moyens de transport (16) de subir tout dommage et tout traumatisme, comprenant des moyens capteurs (12a, 12b, 112a, 112b, 26) en état de détecter toute variation en ce qui concerne notamment les paramètres des conditions d'utilisation desdits moyens de transport (16), **caractérisé en ce qu'**il comprend des moyens de stimulation (15) en état d'être associés à des parties spécifiques du corps des utilisateurs desdits moyens de transport (16) et commandés par lesdits moyens capteurs (12a, 12b, 112a, 112b, 26) en vue d'engendrer des signaux associés pour stimuler lesdites parties du corps de l'utilisateur en réponse à la détection de situations anomales dans les paramètres de fonctionnement desdits moyens de transport (16) telles qu'elles sont détectées par lesdits moyens capteurs (12a, 12b, 112a, 112b, 26).

2. Dispositif d'électrostimulation selon la revendication 1, **caractérisé en ce qu'**il comprend une unité de traitement et de contrôle (13) en état de recevoir et de traiter les signaux transmis par lesdits moyens capteurs (12a, 12b, 112a, 112b, 26) et de conditionner la mise en service desdits moyens de stimulation (15) d'une façon corrélée.

3. Dispositif d'électrostimulation selon la revendication 2, **caractérisé en ce que** ladite unité de traitement et de contrôle (13) est appropriée pour mettre en service lesdits moyens de stimulation (15) moyennant un signal électrique transmis par des câbles et des connecteurs (17, 18).

4. Dispositif d'électrostimulation selon la revendication 2, **caractérisé en ce que** ladite unité de traitement et de contrôle (13) comprend des moyens de transmission (30) pour transmettre des ondes radioélectriques et lesdits moyens de stimulation (15) sont associés à des moyens de réception (31) pour recevoir lesdites ondes radioélectriques.

5. Dispositif d'électrostimulation selon l'une quelconque des revendications précédentes, **caractérisé en ce que** ledit moyen de transport (16) comprend une automobile, un véhicule à moteur, ou tout autre moyen en état de transporter des personnes soit individuellement soit collectivement, tel qu'un train ou un avion, civil ou militaire.

6. Dispositif d'électrostimulation selon l'une quelconque des revendications précédentes, **caractérisé en ce que** ladite situation anomale comprend une variation soudaine de vitesse, soit positive soit négative, ou une inclinaison dudit moyen de transport (16).

7. Dispositif d'électrostimulation selon l'une quelconque des revendications précédentes, **caractérisé en ce que** lesdits moyens de stimulation comprennent au moins un électrode (15) en état d'être appliqué à au moins une partie localisée du corps de l'utilisateur en vue de transmettre au moins une impulsion électrique à ladite partie.

8. Dispositif d'électrostimulation selon la revendication 7, **caractérisé en ce qu'**il y a au moins deux électrodes (15) pour définir au moins une voie correspondante pour transmettre ladite impulsion électrique.

9. Dispositif d'électrostimulation selon la revendication 8, **caractérisé en ce qu'**il y a 16 électrodes ou même plus (15).

10. Dispositif d'électrostimulation selon l'une quelconque des revendications précédentes, **caractérisé en ce que** lesdits moyens de stimulation (15) comprennent une prise individuelle avec deux électrodes.

11. Dispositif d'électrostimulation selon l'une quelconque des revendications précédentes, **caractérisé en ce que** lesdits moyens de stimulation (15) sont distribués de façon à stimuler au moins une partie des muscles paravertébraux de la tête, du cou et des épaules dudit utilisateur.

12. Dispositif d'électrostimulation selon l'une quelconque des revendications précédentes, **caractérisé en ce que** lesdits moyens de stimulation (15) sont distribués de façon à stimuler la partie des muscles de l'utilisateur qui peut influencer le débit de la pression sanguine.

13. Dispositif d'électrostimulation selon l'une quelconque des revendications précédentes, **caractérisé en ce que** lesdits moyens de stimulation (15) sont introduits dans les rembourrages arrière d'un casque de protection (34), dans un collier, dans un siège (19) ou dans des soutiens doux, cachés et en forme de fourchette d'un repose-tête soit dans lesdits moyens de transport (16) soit sur les branches des lunettes dudit utilisateur.

14. Dispositif d'électrostimulation selon l'une quelconque des revendications de 7 à 13, **caractérisé en ce que** ladite impulsion électrique est variable en termes d'amplitude, largeur, fréquence d'application, avec ou sans la présence de l'impulsion négative.

15. Dispositif d'électrostimulation selon la revendication 14, **caractérisé en ce que** ladite impulsion électrique a une différence en termes de potentiel oscillant entre 50 et 250 V, une fréquence oscillant entre 15 et 170 Hz, une largeur oscillant entre 10 et 400 µs et une intensité de courant oscillant entre 1 et 100 mA.

16. Dispositif d'électrostimulation selon l'une quelconque des revendications précédentes, **caractérisé en ce que** lesdits moyens capteurs (12a, 12b, 112a, 112b, 26) sont en état d'être interfacés dans lesdits moyens de transport (16) avec des moyens capteurs associés à un dispositif pour mettre en service au moins un airbag (14) ou au moins un siège éjectable (19).

17. Dispositif d'électrostimulation selon l'une quelconque des revendications précédentes, **caractérisé en ce que** lesdits moyens capteurs (12a, 12b, 112a, 112b, 26) sont en état d'être positionnés soit dans un siège (19) soit sur le tableau de bord d'un véhicule, dans la nacelle ou associés à des membres pour piloter un avion (116).

18. Dispositif d'électrostimulation selon l'une quelconque des revendications précédentes, **caractérisé en ce que** lesdits moyens capteurs (12a, 12b, 112a, 112b, 26) sont en état d'être positionnés dans le siège d'une motocyclette et/ou en association avec un casque de motocycliste.

19. Dispositif d'électrostimulation selon l'une quelconque des revendications de 2 à 18, **caractérisé en ce que** ladite unité de traitement et de contrôle (13) comprend au moins un microprocesseur programmable (21).

20. Dispositif d'électrostimulation selon la revendication 19, **caractérisé en ce que** ledit microprocesseur programmable (21) comprend une matrice logique de traitement (23), un bloc temporisateur (24) et un bloc générateur d'impulsions (25, 27), associés auxdits moyens de stimulation (15).

21. Dispositif d'électrostimulation selon l'une quelconque des revendications de 1 à 6, **caractérisé en ce que** lesdits moyens de stimulation comprennent des moyens d'alimentation en état d'apporter un fluide conducteur vers une ou plusieurs parties du corps de l'utilisateur.

22. Dispositif d'électrostimulation selon la revendication 21, **caractérisé en ce que** ledit fluide conducteur comprend un gaz ou un liquide conducteur, tel que de l'eau salée ou similaire.

23. Dispositif d'électrostimulation selon la revendication 22, **caractérisé en ce que** ledit liquide conducteur est transporté sous une forme nébulisée.

24. Dispositif d'électrostimulation selon la revendication 21, **caractérisé en ce que** lesdits moyens d'alimentation sont aménagés et/ou intégrés dans une partie soit du véhicule soit de l'équipement de l'utilisateur, telle que le siège, le repose-tête, le casque, la caisse, le tableau de bord, ou autres.
